## Europäisches Patentamt

## European Patent Office

## Office européen des brevets

(19)

(11) Veröffentlichungsnummer: **0 092 080**
A1

(12)
# EUROPÄISCHE PATENTANMELDUNG

(21) Anmeldenummer: **83103181.0**

(22) Anmeldetag: **30.03.83**

(51) Int. Cl.³: **A 61 B 10/00**, A 61 B 1/26

(30) Priorität: **21.04.82 DE 3214793**

(43) Veröffentlichungstag der Anmeldung: **26.10.83**
**Patentblatt 83/43**

(84) Benannte Vertragsstaaten: **AT CH DE FR GB LI**

(71) Anmelder: **SIEMENS AKTIENGESELLSCHAFT, Berlin und München Wittelsbacherplatz 2, D-8000 München 2 (DE)**

(72) Erfinder: **Hetz, Walter, Adam-Kraft-Strasse 17, D-8520 Erlangen (DE)**

(54) **Ultraschall-Endoskop.**

(57) Ein Ultraschall-Endoskop (1), insbesondere für die Gastroskopie, besteht aus einer in einen Körper einführbaren Endoskopsonde (5), an deren distalem Ende ein Ultraschallkopf (13) mittels eines Schlauchüberzugs (12) aufgezogen werden kann. Der Schlauchüberzug (12) kann aber auch von der Sonde (5) abgezogen werden, so daß das Endoskop (1) ohne Ultraschallkopf (13) eingesetzt werden kann. Signalleitungen (27) zum Anschluß des Ultraschallkopfes (13) an ein Signalsteuer- und/oder Verarbeitungsgerät verlaufen in der Wand des Schlauchüberzugs (12) bis zum proximalen Schlauchende.

EP 0 092 080 A1

SIEMENS AKTIENGESELLSCHAFT     Unser Zeichen
Berlin und München             VPA 82 P 3735 E

Ultraschall-Endoskop

Die Erfindung bezieht sich auf ein Ultraschall-Endoskop, insbesondere für die Gastroskopie, nach den Merkmalen des Oberbegriffs des Patentanspruchs 1.

Ein Ultraschall-Endoskop dieser Art ist z.B. durch die DE-OS 30 09 482 vorbekannt. Nachteilig ist dabei, daß der Ultraschallkopf ständig mit der Endoskopsonde verbunden ist.

Aufgabe vorliegender Erfindung ist es, ein Endoskop anzugeben, das sowohl mit als auch bei Bedarf ohne Ultraschallkopf einsetzbar ist.

Die Aufgabe wird erfindungsgemäß mit den kennzeichnenden Merkmalen des Anspruchs 1 gelöst.

Gemäß der Erfindung wird also die Endoskopsonde zum Zwecke einer Ultraschalluntersuchung mit dem Schlauch oder Schlauchüberzug überzogen, an dem der Ultraschallkopf sitzt. Der Ultraschallkopf ist also nicht ständig mit der Endoskopsonde verbunden. Vielmehr kann nach Durchführung einer Ultraschalluntersuchung der Ultraschallkopf durch Abziehen des Schlauches von der Endoskopsonde getrennt werden. Dann kann das Endoskop ohne Ultraschallkopf für normale Endoskopieuntersuchungen eingesetzt werden.

Als Vorteil wird es angesehen, daß - basierend auf dem hier angegebenen Prinzip - ein bereits vorhandenes

Nm 2 Rl / 16.02.1983

**0092080**

Endoskop mit einer Ultraschall-Zusatzeinrichtung nachgerüstet werden kann. Dies ist verhältnismäßig einfach durchzuführen. Ein solches Nachrüsten kann direkt beim Anwender, also z.B. beim Arzt im Krankenhaus, ausge-führt werden.

Eine Ultraschall-Prüfsonde, bei der ein Ultraschallkopf bereits am distalen Ende eines Sondenschlauches angeordnet ist, ist an sich aus dem DE-GM 69 42 159 vorbekannt. Der betreffende Sondenschlauch ist aber kein Schlauchüberzug im Sinne der Erfindung, der also z.B. über eine Endoskopsonde ziehbar ist, so daß der Ultraschallkopf am distalen Sondenende zum Sitzen kommt. Der bekannte Sondenschlauch trägt vielmehr in seinem Innern Spiralfedern sowie ein koaxiales Verbindungskabel mit den Signalleitungen des Ultraschallkopfes. Die Signalleitungen des Ultraschallkopfes sind dabei durch das hohle Innere des Sondenschlauches und nicht, wie es vorliegende Erfindung fordert, eingebettet in der Schlauchwandung geführt.

Durch die DE-PS 23 05 501 ist schließlich noch eine Ultraschallsonde vorbekannt, bei der der Ultraschallkopf an einem starren Sondenrohr gehaltert ist. Auch dieses Sondenrohr dient nicht als Schlauchüberzug im Sinne der vorliegenden Erfindung.

Weitere Vorteile und Einzelheiten der Erfindung ergeben sich aus der nachfolgenden Beschreibung eines Ausführungsbeispiels anhand der Zeichnung und in Verbindung mit den Unteransprüchen.

Es zeigen:

Fig. 1 ein übliches Endoskop, insbesondere für Gastroskopie, ohne Ultraschallzusatz,

Fig. 2 das distale Kopfende einer Endoskopsonde gemäß Figur 1 in Vergrößerung,

Fig. 3 ein in ein Ultraschall-Endoskop gemäß der
Erfindung umgewandeltes normales Endoskop
entsprechend Figur 1,

Fig. 4 das distale Ende des Ultraschallzusatzes in
Seitenansicht, teilweise im Schnitt,

Fig. 5 eine Kopfansicht des distalen Endes der
Endoskopsonde mit Ultraschallzusatz,

Fig. 6 eine Draufsicht auf das distale Ende der
Endoskopsonde mit Ultraschallzusatz,

Fig. 7 bis 10 Montageteile für das distale Ende des
Ultraschallzusatzes,

Fig. 11 die proximale Ankopplung des Ultraschallzusatzes am Endoskop,

Fig. 12 ein weiteres Endoskop in Schnittdarstellung.

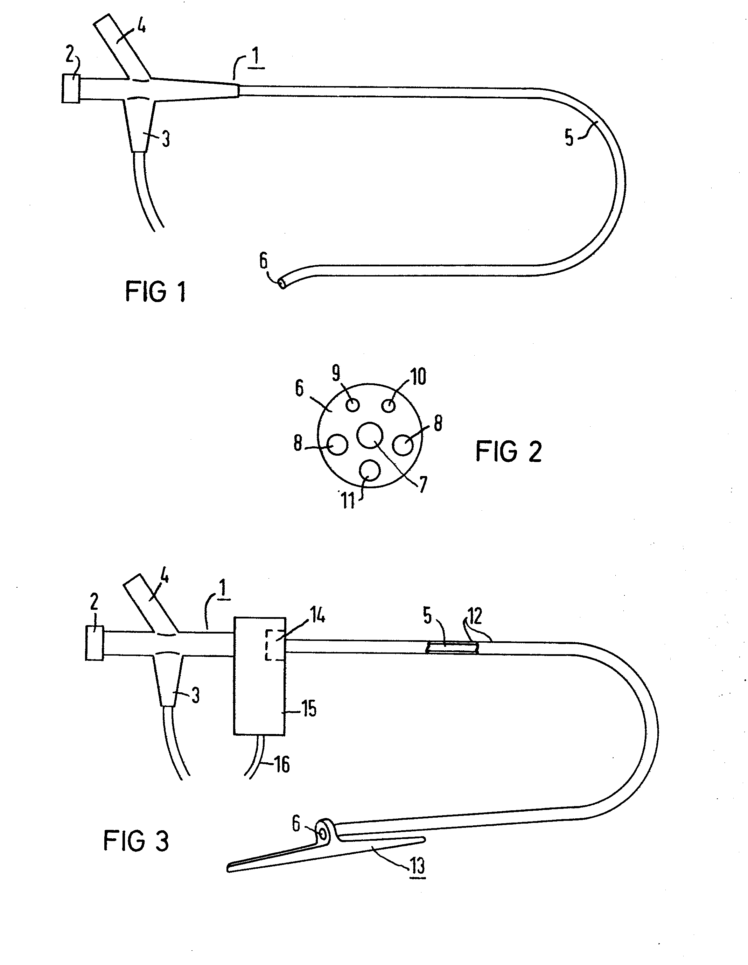

Die Figur 1 zeigt ein Endoskop 1, das proximal mit einem Okular 2, einem Kaltlichtanschluß 3 und einem Einlaß 4 für einen Arbeitskanal sowie distal mit einer
flexiblen, länglichen Endoskopsonde 5 bestückt ist. Das
Endoskop 1 der Figur 1 dient insbesondere für Gastroskopiezwecke, d.h. die Endoskopsonde 5 wird über die
Speiseröhre in den Magen eines Patienten zu dessen Untersuchung eingeführt. Das Kopfende 6 der Endoskopsonde
5 ist in der Figur 2 in Vergrößerung dargestellt. Demgemäß münden also am Kopfende 6 in herkömmlicher Weise
die Untersuchungsoptik 7 zusammen mit zwei Glasfiberbündeln 8 für die Beleuchtung, ein Düsenkanal 9 für
Spülwasser, ein Kanal 10 für Druckluft und ein Arbeitskanal 11 für Resektion, Absaugen oder Biopsie. Insoweit handelt es sich also bei dem Endoskop 1 der Figuren 1 und 2 um ein übliches Gastroskop.

Die Figur 3 zeigt dasselbe Endoskop, diesmal jedoch mit Ultraschallzusatz. Der Ultraschallzusatz besteht dabei aus einem Schlauch oder Schlauchüberzug 12, an dessen distalem Ende ein langgestrecktes Ultraschall-Array 13 angeordnet ist. Dessen Längsrichtung erstreckt sich dabei in Richtung der Endoskopsonde 5. Der Schlauchüberzug 12 ist hier auf die Endoskopsonde 5 aufgeschoben. Bei Bedarf kann der Schlauchüberzug 12 wieder von der Endoskopsonde 5 abgezogen und das Ultraschall-Array 13 kann entfernt werden, so daß konventionell endoskopiert werden kann. Die Signalleitungen des Arrays 13 verlaufen eingebettet in der Wandung des Schlauches 12, und zwar vom Array 13 aus bis zu einem proximalen Anschlußteil 14. Über dieses Anschlußteil 14 wird die elektrische Verbindung zu einem Elektronikteil 15 als Bestandteil eines Signalsteuer- und Signalverarbeitungsgerätes für die Ultraschallsignale des Ultraschall-Arrays 13 hergestellt. Der Elektronikteil 15 ist am proximalen Teil des Endoskops 1 angeordnet, z.B. aufgeschoben. Ein Signalkabel 16 verbindet den Elektronikteil 15 mit einem externen (nicht dargestellten) Ultraschallgerät für Energieversorgung und Bildverarbeitung oder Bildanzeige der Ultraschall-Echosignale.

Der Schlauchüberzug 12 besteht vorzugsweise aus Silikonkautschuk. In die Wand des Schlauchüberzugs 12 sind etwa fünfzig HF-abgeschirmte Signalleitungen eingebettet.Freie Zwischenräume zwischen den Leitungen sind mit Silikonkautschukkleber ausgefüllt.

Anstelle eines langgestreckten Ultraschall-Arrays 13 kann auch ein verhältnismäßig kurzer Ultraschallkopf für Sektor-Abtastung eingesetzt werden.

0092080

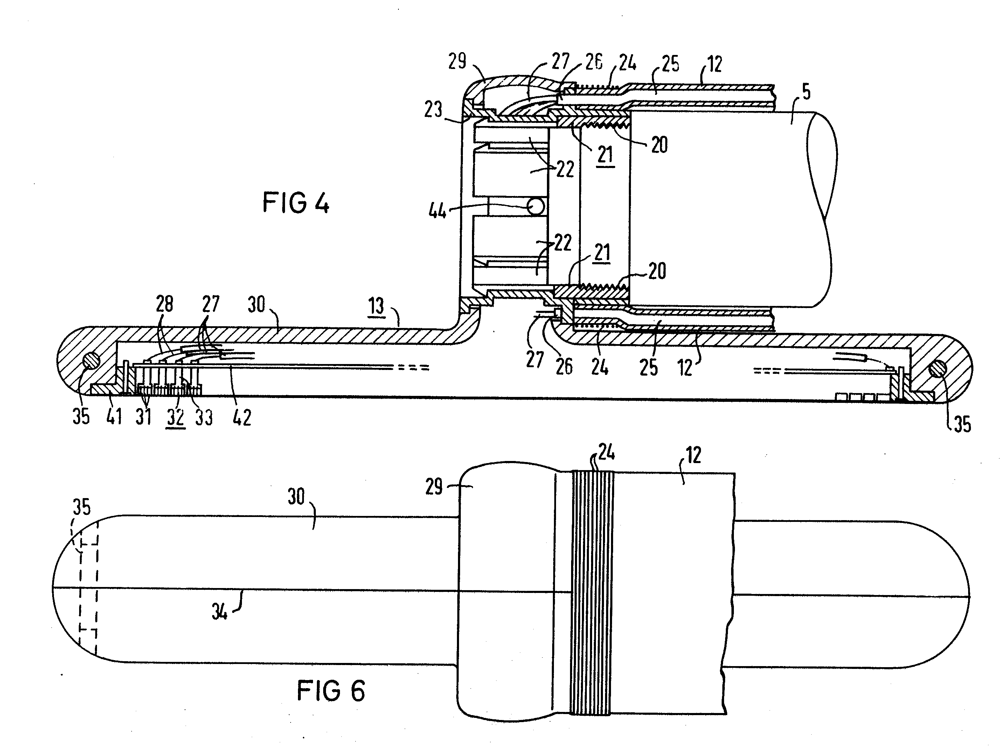

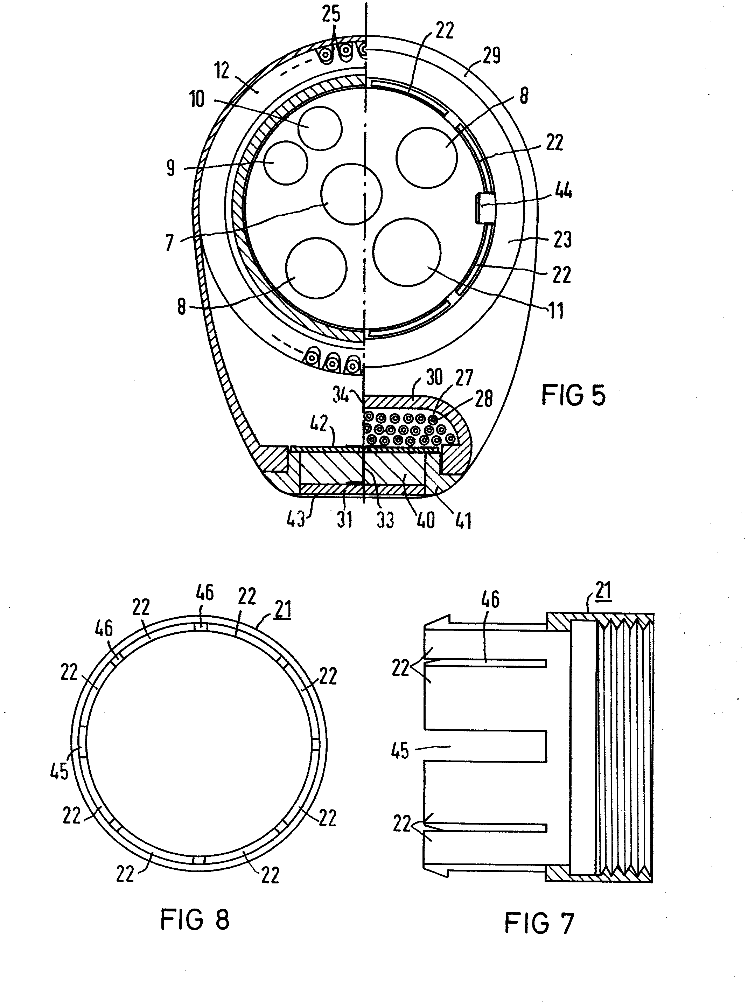

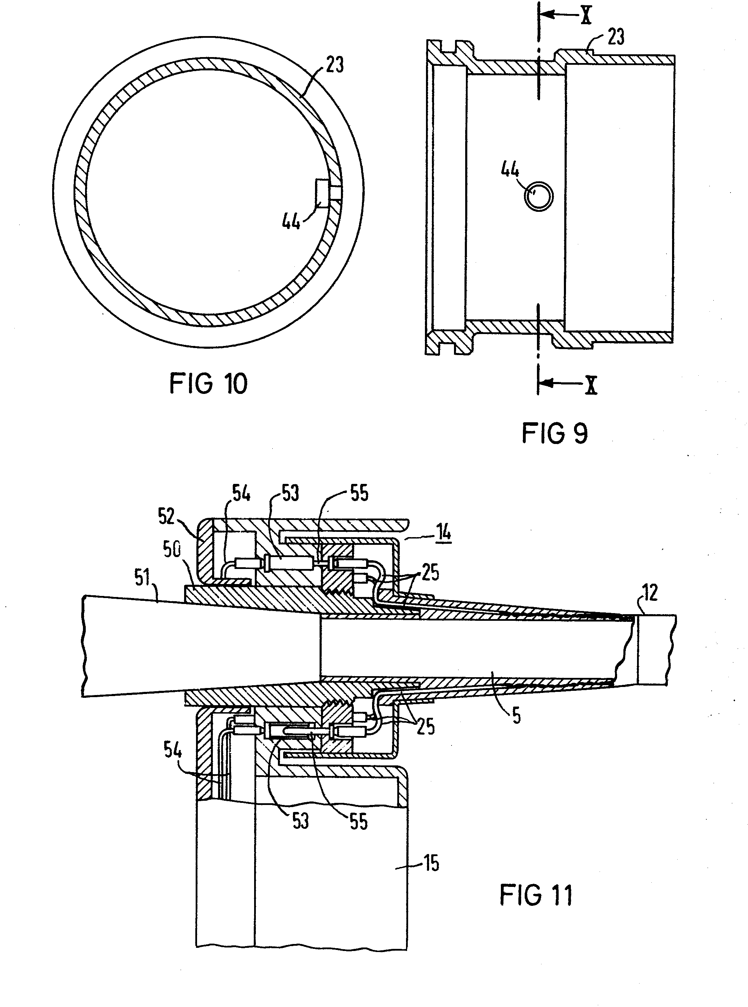

Die Figur 4 zeigt in Vergrößerung das distale Ende der Endoskopsonde 5. Auf ein Außengewinde 20 des distalen Endes ist eine Schnapphülse 21, z.B. aus Federbronze, die Federzungen 22 umfaßt, aufgeschraubt. Der Aufbau der Schnapphülse ist in den Figuren 7 und 8 in Seiten- und Frontansicht näher dargestellt. Auf der Schnapphülse 21 liegt eine Schlauchhülse 23, die im Detail in den Figuren 9 und 10 in Seiten- und Querschnittansicht erläutert ist. Die Federzungen 22 greifen in eine Schulter ein, die im Innern der Schlauchhülse 23 angeordnet ist. Wie später deutlich werden wird, fixieren die Federzungen 22 den Ultraschallkopf 13 in einer lösbaren Weise. An der Schlauchhülse 23 ist das distale Ende des Schlauchüberzugs 12 mittels eines Schlingfadens 24 befestigt. Im Innern des Schlauchüberzugs 12 liegen die Signalleitungen 25 mit der HF-Abschirmung 26, der Innenkabelisolierung 27 und den Kontaktdrähten 28. Die isolierten Kontaktdrähte 28 werden durch das Innere eines Ringgehäuses 29, das ein Array-Gehäuse 30 trägt, in das Innere des Array-Gehäuse 30 zu den einzelnen Wandlerelementen des Arrays 13 zwecks Kontaktierung geführt.

In der Figur 4 sind die Wandlerelemente mit 31 bezeichnet. Es handelt sich um feingeteilte Wandlerelemente, von denen jeweils immer z.B. vier Stück zu einer Gruppe 32 (von z.B. insgesamt 48 Gruppen) elektrisch zusammenkontaktiert sind. Zur Zusammenkontaktierung dient ein Kontaktstreifen 33. Das Prinzip der Feinteilung und der Gruppenkontaktierung ist z.B. aus der DE-PS 28 29 570 vorbekannt.

In einer praktischen Ausführung bestehen der Haltering 29 und das Array-Gehäuse 30 aus zwei Metallhalbschalen, die, wie in der Figur 6 dargestellt, entlang der Stoßlinie 34 miteinander verklebt werden. Zum Einpassen der
Halbschalen dienen zwei Paßstifte 35. Die Halbschalen
sind aus Metall, z.B. rostfreiem Stahl, gefertigt. Sie
können nach Montage mit einem Isolierstoff, z.B. einer
Kappe aus Isoliergummi, überzogen werden.

Die Figur 5 zeigt eine Kopfansicht der Endoskopsonde
mit Ultraschallzusatz. Die Bezugsziffern sind dieselben
wie im Ausführungsbeispiel der Figur 4. Im Array 13
dient als Träger für die Wandlerelemente 31 ein Trägerkörper 40, der z.B. aus Epoxydharz mit eingebrachtem
oxidiertem Wolframpulver besteht. Trägerkörper 40 und
Wandlerelemente 31 sind mittels Montagestücken 41 im
Array-Gehäuse 30 montiert. Das Bauteil 42 ist eine
Leiterplatte. Diese kann bei Bedarf auch wegfallen.
Die Applikationsfläche des Arrays 13 ist mit einer
Anpassungsschicht 43 für die Wandlerelemente 31 versehen.

Um dem Array 13 bezüglich der Kanäle 7 bis 11 des
Kopfes der Endoskopsonde 5 eine bestimmte Winkellage
zu geben, ist die Schlauchhülse 23 mit einem besonderen
Arretierstift 44 versehen. Der Arretierstift 44 ist, wie
in den Figuren 9 und 10 angedeutet, in die Schlauchhülse
23 eingelötet. Bei der Montage muß darauf geachtet werden, daß der Arretierstift 44 in einen dazu passenden
breiteren Schlitz 45 (siehe Figuren 7 und 8) zwischen
zwei Federzungen 22 der Schnapphülse 21 einrastet. Die
restlichen Schlitze zwischen den Federzungen 22 der
Schnapphülse 21 sind weniger breit als der Schlitz 45.
In den Figuren 7 und 8 sind einige der schmaleren
Schlitze mit 46 bezeichnet.

Die Figur 11 zeigt den proximalen Anschluß des Schlauchüberzugs 12. Das proximale Schlauchende ist dabei kopfseitig mit einer Überschiebehülse 50 versehen, die auf das konische Endteil 51 des Endoskops 1 aufschiebbar ist, das in die Endoskopsonde 5 übergeht. Auf der Überschiebhülse 50 sitzt mittels eines Überschieberinges 52 der an diesem gehalterte Elektronikteil 15. Der Überschiebering 52 hat die in der Figur 11 im Schnitt dargestellte Ausbildung. Er enthält demnach in seinem Innern eine kreisförmige Anordnung von Steckbuchsen 53 mit Leitungen 54. Zu diesen Steckbuchsen 53 passen Steckstifte 55. Diese Steckstifte 55 sind in entsprechender kreisförmiger Anordnung Bestandteil des Endsteckers 14 zur Ankopplung der proximalen Enden der Signalleitungen 25 an das Elektronikteil 15.

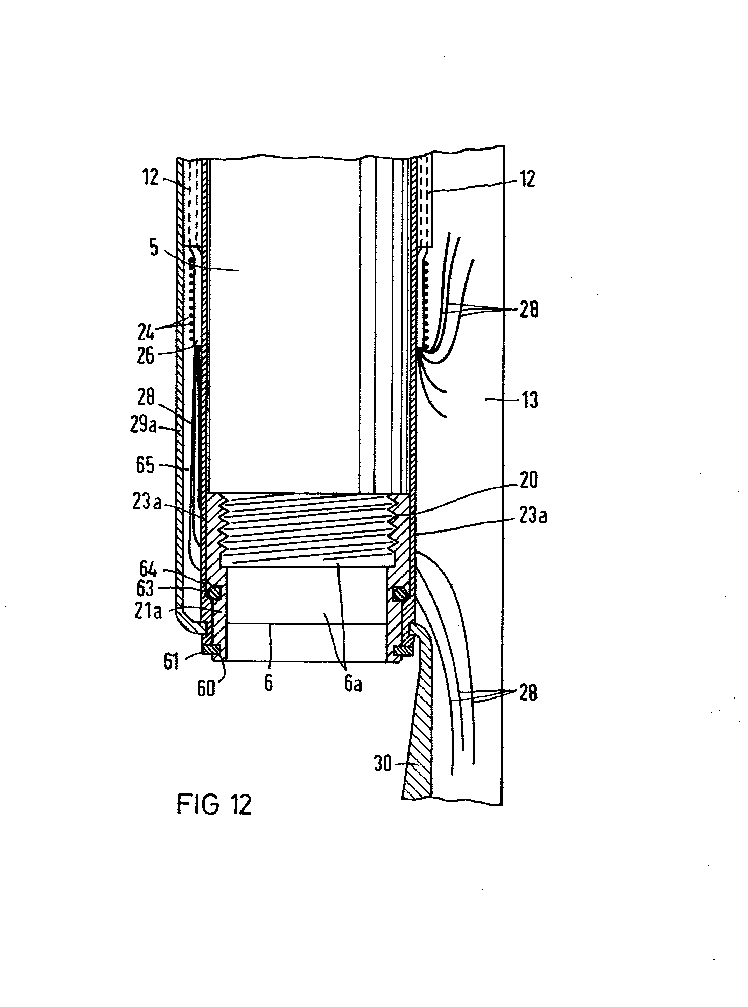

In Figur 12 ist ein weiteres bevorzugtes Ausführungsbeispiel eines Ultraschall-Endoskops dargestellt. Diese Ausführungsform ist eine Modifikation des Ausführungsbeispieles nach Figur 4. Figur 12 stellt dabei einen Schnitt durch das Endteil des Endoskops dar. Gleiche und entsprechende Teile sind dabei mit denselben Bezugszeichen versehen wie in Figur 3.

Die in Figur 12 gezeigte Ausführungsform kommt ohne einen Schnappring aus. Bei dieser Ausführungsform enthält die Endoskopsonde 5 wiederum an ihrem distalen Ende ein Endstück 6a. Dieses Endstück 6a ist im wesentlichen ein zylindrisches Metallstück konventioneller Bauart, welches die in Figur 2 dargestellte Endfläche aufweist. Das Endstück 6a besitzt ein Außengewinde 20, auf das ein zylindrisches Paßstück 21a in Form eines Rohres aufgeschraubt ist. Dieses Aufschraubstück 21a erstreckt sich mit seinem Ende einiges über die Endfläche 6 hinaus. Das distale Ende dieses Paßstückes 21a enthält dabei eine ringförmige Rille 60. Ein Rückhaltring 61 ist in die Rille 60 eingeschnappt. Das Paßstück 21a erfüllt im wesentlichen die Funktion des in Figur 3 dargestellten Schnappsockels 21.

Auf die Endoskopsonde 5 und auf das Paßstück 21a ist ein längeres Rohr 23a aufgebracht. Dieses entspricht in seiner Wirkung dem Rohrsockel 23 nach Figur 3. Die Lage dieses Rohres 23a ist mit Hilfe des zuvor erwähnten Schnappringes 61 festgelegt. Um zu verhindern, daß Flüssigkeit in den Zwischenraum zwischen der Endoskopsonde 5 und dem Rohr 23 eindringen kann, ist ein O-Ring 63 vorgesehen. Dieser O-Ring 63 ist zwischen dem Paßstück 21a und dem Endteil des Rohres 23a angeordnet. Der O-Ring 63 befindet sich in einer ringförmigen Rille 63, welche in die äußere Oberfläche des Paßstückes 21a eingelassen ist. Der O-Ring 63 verschließt somit das Innere des Rohres 23a. Dieses ist von Bedeutung, denn das Endteil des Gastroskops ist während des Gebrauches oder insbesondere während des Spülens der Optik Flüssigkeiten ausgesetzt.

Das Rohr 23a trägt ein langgestrecktes Gehäuse 29a, welches in das Gehäuse 30 des Ultraschallkopfes 13 einmündet. Das gesamte Gehäuse 29a, 30 ist vorzugsweise aus einem Kunststoff hergestellt. Es ist bemerkenswert, daß ein Zwischenraum 65 zwischen der äußeren Oberfläche des Rohres 23a und der inneren Oberfläche des Gehäuses 29a vorgesehen ist.

Ein Schlauchstück 12 ist innerhalb dieses Zwischenraumes 65 untergebracht. Das Schlauchstück 12 selbst umfaßt eine Abschirmung 26, welche eine größere Anzahl von Zuleitungsdrähten 28 enthält, die zum Ultraschall-Übertrager 13 führen. Die Abschirmung 26 wird mittels eines Drahtes 24 festgehalten, der sowohl um das Rohr 23a als auch um die Abschirmung 26 herumgewickelt ist. Die Drähte 28 sind elektrische Signalverbindungen, welche den Ultraschall-Übertrager 13 an eine äußere Steuer- und Verarbeitungseinheit (nicht gezeigt) anschließen.

Somit ist also auch in der Ausführungsform nach Figur 12

0092080

das Schlauchstück 12 über die Endoskopsonde 6 gestülpt. Die elektrischen Signalleitungen 28 sind in der Schlauchwand untergebracht.

21 Patentansprüche
12 Figuren

Patentansprüche

1. Ultraschall-Endoskop, insbesondere für Gastroskopie, mit einer in einen Körper, z.B. Patientenkörper, einführbaren Endoskopsonde, an deren distalem Sondenende ein Ultraschallkopf sitzt, der über Signalleitungen, die entlang der Endoskopsonde zum proximalen Sondenende geführt sind, mit einem Signalsteuer- und/oder Verarbeitungsgerät verbindbar ist, d a d u r c h  g e - k e n n z e i c h n e t , daß der Ultraschallkopf (13) am einen Ende eines Schlauchüberzuges (12) angeordnet ist, welcher Schlauchüberzug (12) auf die Endoskopsonde (5) aufschiebbar ist, so daß der Ultraschallkopf (13) am besagten distalen Sondenende sitzt, und welcher Schlauchüberzug (12) wieder von der Endoskopsonde (5) abziehbar ist, und daß die Signalleitungen (25) des Ultraschallkopfes (13) eingebettet in die Wand des Schlauchüberzuges (12) sind und vom einen Schlauchende am Ort des Ultraschallkopfes (13) bis zum anderen Schlauchende verlaufen.

2. Ultraschall-Endoskop nach Anspruch 1, d a d u r c h  g e k e n n z e i c h n e t , daß der Ultraschallkopf (13) am distalen Ende des Schlauchüberzuges (12) so angeordnet ist, daß die Arbeitsstirnfläche (6) der Endoskopsonde (5) frei bleibt.

3. Ultraschall-Endoskop nach Anspruch 1 oder 2, d a - d u r c h  g e k e n n z e i c h n e t , daß eine Einrichtung (21, 23, 29) vorgesehen ist, um den Ultraschallkopf (13) am distalen Ende des Schlauchüberzugs (12) lösbar zu befestigen.

4. Ultraschall-Endoskop nach Anspruch 3, d a d u r c h  g e k e n n z e i c h n e t , daß das distale Ende

0092080

der Endoskopsonde (5) eine Schnapphülse (21) trägt, die Federzungen (22) umfaßt, daß auf der Schnapphülse (21) eine Schlauchhülse (23) als distales Endstück für den Schlauchüberzug (12) liegt, und daß die Federzungen (22) in eine Schulter in der Schlauchhülse (23) eingreifen.

5. Ultraschall-Endoskop nach Anspruch 4, d a - d u r c h   g e k e n n z e i c h n e t ,   daß das distale Ende des Schlauchüberzuges (12) auf der Schlauchhülse (23) mittels eines Schlingfadens (24) befestigt ist.

6. Ultraschall-Endoskop nach Anspruch 4 oder 5, d a - d u r c h   g e k e n n z e i c h n e t ,   daß die Schnapphülse (21) auf ein Gewinde (20), das am distalen Ende der Endoskopsonde (5) angebracht ist, aufgeschraubt ist.

7. Ultraschall-Endoskop nach einem der Ansprüche 4 bis 6, d a d u r c h   g e k e n n z e i c h n e t ,   daß die Schlauchhülse (23) mit wenigstens einem Arretierstift (44) versehen ist, der in einen breiteren Schlitz (45) zwischen zwei Federzungen (22) der Schnapphülse (21) einrastet.

8. Ultraschall-Endoskop nach Anspruch 7, d a d u r c h g e k e n n z e i c h n e t ,   daß die weiteren Schlitze (46) zwischen den Federzungen (22) der Schnapphülse (21) weniger breit sind als der breitere Schlitz (45) für den Arretierstift (44).

9. Ultraschall-Endoskop nach einem der Ansprüche 4 bis 8, d a d u r c h   g e k e n n z e i c h n e t ,   daß der Ultraschallkopf (13) mittels eines Halterings (29) auf der Schlauchhülse (23) befestigt ist.

0092080

10. Ultraschall-Endoskop nach Anspruch 9, d a d u r c h
g e k e n n z e i c h n e t , daß das Schallkopfgehäuse (30) und der Haltering (29) aus zwei Halbschalen
bestehen, die nach Montage auf der Schlauchhülse (23).
entlang einer Stoßlinie (34) miteinander verbunden,
vorzugsweise miteinander verklebt, sind.

11. Ultraschall-Endoskop nach Anspruch 10, d a -
d u r c h g e k e n n z e i c h n e t , daß die
Halbschalen zum Einpassen mit Paßstiften (35) und
dazu passenden Paßbohrungen versehen sind.

12. Ultraschall-Endoskop nach Anspruch 10 oder 11,
d a d u r c h g e k e n n z e i c h n e t , daß
die Halbschalen aus nicht rostendem Material, z.B.
rostfreiem Stahl, gefertigt sind, wobei nach der Montage der Ultraschallkopf (13) mit einem Isolierstoff,
z.B. einer Kappe aus Isoliergummi, überzogen werden
kann.

13. Ultraschall-Endoskop nach einem der Ansprüche 1
bis 12, d a d u r c h g e k e n n z e i c h n e t ,
daß der Schlauchüberzug (12) aus Silikonkautschuk besteht.

14. Ultraschall-Endoskop nach Anspruch 13, d a -
d u r c h g e k e n n z e i c h n e t , daß in
der Wand des Schlauchüberzugs (12) gegen Hochfrequenzeinstrahlung abgeschirmte Signalleitungen (25) eingebettet sind.

15. Ultraschall-Endoskop nach Anspruch 14 oder 15,
d a d u r c h g e k e n n z e i c h n e t , daß
die Zwischenräume zwischen den Signalleitungen (25)
in der Wand des Schlauchüberzugs (12) mit Silikonkautschukkleber ausgefüllt sind.

16. Ultraschall-Endoskop nach einem der Ansprüche 1 bis 15, d a d u r c h g e k e n n z e i c h n e t , daß auf einem Endteil (51) des Endoskops, wo das Endoskop in die Endoskopsonde (5) übergeht, ein Elektronikteil (15) als Bestandteil eines Signalsteuer- und Signalverarbeitungsgerätes sitzt, der Kontakte (53) umfaßt, zu denen Gegenkontakte (55) eines Endsteckers (14) passen, der am proximalen Ende des Schlauchüberzugs (12) sitzt und der der Endstecker für die proximalen Enden der Signalleitungen (25) ist.

17. Ultraschall-Endoskop nach Anspruch 16, d a d u r c h g e k e n n z e i c h n e t , daß der Elektronikteil (15) kopfseitig mit einem Überschiebring (52) versehen ist, der auf das Endteil (51) des Endoskops aufschiebbar ist, das in die Endoskopsonde (5) übergeht.

18. Ultraschall-Endoskop nach Anspruch 17, d a d u r c h g e k e n n z e i c h n e t , daß auch der Schlauchüberzug (12) proximal mit einer Überschiebhülse (50) versehen ist, die zuerst auf das Endteil (51) des Endoskops aufschiebbar ist und auf die dann der Überschiebring (50) des Elektronikteils (15) schiebbar ist.

19. Ultraschall-Endoskop nach einem der Ansprüche 16 bis 18, d a d u r c h g e k e n n z e i c h n e t , daß der Elektronikteil (15) in einer Ausbuchtung des Gehäuses eine kreisförmige Anordnung von weiblichen Steckbuchsen (53) mit Fortführleitungen (54) umfaßt, und daß zu diesen weiblichen Steckbuchsen männliche Steckstifte (55) passen, die in entsprechender kreisförmiger Anordnung Bestandteil des Endsteckers (14) zur Ankopplung der proximalen Enden der Signalleitung (25) an dem Elektronikteil (15) sind.

20. Ultraschall-Endoskop nach einem der Ansprüche 1 bis 19, d a d u r c h  g e k e n n z e i c h n e t , daß der Ultraschallkopf (13) ein Ultraschall-Array, vorzugsweise ein feingeteiltes Ultraschall-Array, ist.

21. Ultraschall-Endoskop nach Anspruch 20,  d a - d u r c h  g e k e n n z e i c h n e t ,  daß der Ultraschallkopf (13) langgestreckt ausgebildet und so am distalen Schlauchende befestigt ist, daß seine Längsachse parallel zur Längsachse der Endoskopsonde (5) liegt.

FIG 1

FIG 2

FIG 3

FIG 4

FIG 6

0092080
82 P 3735

FIG 5

FIG 8

FIG 7

0092080

82 P 3735

FIG 10

FIG 9

FIG 11

FIG 12

Europäisches
Patentamt

**EUROPÄISCHER RECHERCHENBERICHT**

0092080

Nummer der Anmeldung

EP 83 10 3181

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| Y | DE-A-2 736 102 (YEDA RESEARCH AND DEVELOPMENT CO., LTD.) * Seite 5, Zeilen 8-31; Seite 8, Zeilen 13-25; Seite 10, Zeilen 7-29; Seite 11, Zeilen 15-27; Seite 13, Zeilen 12-24; Abbildungen 1-4 * | 1,19, 20 | A 61 B 10/00 A 61 B 1/26 |
| Y | US-A-4 319 580 (P.S. COLLEY et al.) * Zusammenfassung; Spalte 5, Zeile 58 - Spalte 6, Zeile 23; Spalte 8, Zeile 6 - Spalte 9, Zeile 30; Abbildungen 3,4,7,8 * | 1-3,9, 14,21 | |
| E | EP-A-0 077 923 (SIEMENS AG.) * Zusammenfassung; Seite 4, Zeile 12 - Seite 7, Zeile 2; Seite 8, Zeilen 5-20; Abbildungen 1-4 * | 1-3,9, 14-17, 19-21 | |
| A | IEEE TRANSACTIONS ON SONICS AND ULTRASONICS, Band SU-27, Nr. 6, November 1980, Seiten 277-286, IEEE, New York, USA R.W. MARTIN et al.: "An ultrasonic catheter for intravascular measurement of blood flow: Technical details" * Seiten 279-281, Abschnitt "General"; Abbildungen 3,5 * | 1,14, 16-21 | |

RECHERCHIERTE SACHGEBIETE (Int. Cl. ³)

A 61 B 10/00
A 61 B 1/00
A 61 B 1/26
A 61 B 1/30
A 61 B 19/04

--- -/-

Der vorliegende Recherchenbericht wurde für alle Patentansprüche erstellt.

| Recherchenort DEN HAAG | Abschlußdatum der Recherche 15-07-1983 | Prüfer RIEB D.K. |
|---|---|---|

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A,D | DE-A-2 305 501 (SIEMENS AG.) <br> * Seite 3, Zeilen 6-18; Seite 4, Zeilen 10-25; Seite 5, Zeilen 18-28; Abbildungen 1-3 * | 1,20 | |
| A | FR-A-2 242 060 (AKADEMIET FOR DE TEKNISKE VIDENSKABER) <br> * Seite 1, Zeile 24 - Seite 2, Zeile 11; Seite 2, Zeilen 33-40; Seite 4, Zeilen 10-19; Abbildungen 1-5 * | 1 | |
| A | US-A-3 315 663 (H. GOLDFARB) <br> * Spalte 2, Zeilen 1-16, 29-51; Spalte 3, Zeilen 11-15; Abbildungen 1-5 * | 1,2 | |
| A | DE-C- 606 098 (E. KALPEN) <br> * Seite 2, Zeilen 10-15, 33-40; Abbildungen 1-5 * | 1,9 | RECHERCHIERTE SACHGEBIETE (Int. Cl. ³) |
| A | DE-B-1 211 360 (A.H. SAEED) <br> * Spalte 3, Zeilen 12-44; Abbildungen 1,2 * | 1,19 | |
| A | EP-A-0 027 185 (OLYMPUS OPTICAL CO., LTD.) <br> * Seite 6, Zeilen 1-24; Abbildung 2 * | 5,13 | |

--- -/-

Der vorliegende Recherchenbericht wurde für alle Patentansprüche erstellt.

| Recherchenort <br> DEN HAAG | Abschlußdatum der Recherche <br> 15-07-1983 | Prüfer <br> RIEB D.K. |
|---|---|---|

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| A | DE-A-2 626 179 (K. STORZ) <br> * Seite 7, Zeilen 5-14; Abbildungen 1-4 * <br><br> --- | 10 | |
| A | US-A-3 556 079 (H. OMIZU) <br> * Spalte 1, Zeilen 57-65; Spalte 3, Zeile 70 - Spalte 4, Zeile 25; Abbildungen 1-6 * <br><br> ----- | 1 | |

RECHERCHIERTE SACHGEBIETE (Int. Cl. 3)

Der vorliegende Recherchenbericht wurde für alle Patentansprüche erstellt.

| Recherchenort <br> DEN HAAG | Abschlußdatum der Recherche <br> 15-07-1983 | Prüfer <br> RIEB D.K. |
|---|---|---|